# EUROPEAN PATENT APPLICATION

(11) **EP 0 890 645 A2**
(43) Date of publication of application: **13.01.1999**
(21) Application number: 98305422.2
(22) Date of filing: 08.07.1998
(51) Int. Cl.: C12N 15/54, C12N 9/10

(54) **Sialyltransferase and DNA encoding the same**

(30) Priority: 09.07.1997 JP 184184/97
(71) Applicant: SEIKAGAKU KOGYO KABUSHIKI KAISHA, Tokyo 103 (JP)
(72) Inventor: Saito, Masaki, Tokyo (JP)
(74) Representative: Bannerman, David Gardner

(57) **Abstract**

A sialyltransferase having the following physico-chemical properties:
(1) Activity:
   transfers sialic acid from a sialic acid donor selectively to a 3-hydroxyl group of a galactose residue contained in lactosylceramide as a sialic acid acceptor to produce ganglioside G_{M3};
(2) Optimal reaction pH:
   6.0 to 7.0; and
(3) Inhibition and activation:
   the activity increases at least 1.5 times with 10 mM of Mn²⁺ as compared with the case in the absence thereof.

### Annex to the description

Sequence Listing

<110> Seikagaku Corporation

<120> Sialyltransferase and DNA encoding the same

<150> JP 9-184184

<151> 1997-07-09

<160> 6

<210> 1

<211> 2121

<212> DNA

<213> Homo sapiens

<220>

<221> CDS

<222> 202..1278

<220>

<221> misc_feature

<222> 247..288

<223> transmembrane domain

<220>

<221> misc_feature

<222> 871..879

<223> potential N-glycosylation site

<220>

<221> misc_feature

<222> 1201..1209

<223> potential N-glycosylation site

<220>

<221> misc_feature

<222> 616..750

<223> sialyl-motif

<220>

<221> misc_feature

<222> 1048..1116

<223> sialyl-motif

<400> 1

cccgggctgg cggcttgcca gcgctccctc cctagcatgc acacagaggc ggtgggcggc 60

gcggcgcgga ggccccagaa gctgcgaagc caagcagcgg cacctgcctg ccgagcaatg 120

ccaagtgagt tcacctctgc aaagctgaga agtgattgct caaggacctc cctgcaatgg 180

tacacccgaa ctcagcacaa g atg aga aga ccc agc ttg tta ata aaa gac 231
Met Arg Arg Pro Ser Leu Leu Ile Lys Asp
1               5                   10

atc tgc aag tgc acg ttg gtt gca ttt gga gtc tgg ctc ctg tac atc 279
Ile Cys Lys Cys Thr Leu Val Ala Phe Gly Val Trp Leu Leu Tyr Ile
                15                  20                  25

ctc att ttg aat tac acc gct gaa gaa tgt gac atg aaa aga atg cac 327
Leu Ile Leu Asn Tyr Thr Ala Glu Glu Cys Asp Met Lys Arg Met His
            30                  35                  40

tat gtg gac cct gac cgg ata aag aga gct cag agc tat gct cag gaa 375
Tyr Val Asp Pro Asp Arg Ile Lys Arg Ala Gln Ser Tyr Ala Gln Glu
        45                  50                  55

gtc ttg cag aag gaa tgt cgg ccc agg tac gcg aag acg gct atg gct 423
Val Leu Gln Lys Glu Cys Arg Pro Arg Tyr Ala Lys Thr Ala Met Ala
    60                  65                  70

ctg tta ttt gag gac agg tac agc atc aac ttg gag cct ttt gtg cag 471
Leu Leu Phe Glu Asp Arg Tyr Ser Ile Asn Leu Glu Pro Phe Val Gln
75                  80                  85                  90

aag gtc ccc acg gcc agt gaa gct gag ctc aag tat gac ccg cct ttt 519
Lys Val Pro Thr Ala Ser Glu Ala Glu Leu Lys Tyr Asp Pro Pro Phe
                95                  100                 105

gga ttc cgg aag ttc tcc agt aaa gtc cag agc ctc ttg gat atg ctg 567
Gly Phe Arg Lys Phe Ser Ser Lys Val Gln Ser Leu Leu Asp Met Leu
            110                 115                 120

ccc gaa cat gac ttt cct gaa cac ttg aga gcc aag gcc tgc aag cgc 615
Pro Glu His Asp Phe Pro Glu His Leu Arg Ala Lys Ala Cys Lys Arg
        125                 130                 135

tgt gtg gtt gtt ggg aac ggg ggc atc ctg cac gga cta gag ctg ggt 663
Cys Val Val Val Gly Asn Gly Gly Ile Leu His Gly Leu Glu Leu Gly
    140                 145                 150

cac gcc ctc aac cag ttc gat gtg gta ata agg ttg aac agt gcg cca 711
His Ala Leu Asn Gln Phe Asp Val Val Ile Arg Leu Asn Ser Ala Pro
155                 160                 165                 170

gtt gag ggt tac tct gaa cac gtt ggg aat aaa act act ata agg atg 759
Val Glu Gly Tyr Ser Glu His Val Gly Asn Lys Thr Thr Ile Arg Met
                175                 180                 185

act tac cca gag ggt gcg cca ctg tcg gac gtt gaa tac tac gcc aat 807
Thr Tyr Pro Glu Gly Ala Pro Leu Ser Asp Val Glu Tyr Tyr Ala Asn
            190                 195                 200

gat ttg ttc gtt act gtt tta ttt aag agt gtt gat ttc aag tgg ctt 855
Asp Leu Phe Val Thr Val Leu Phe Lys Ser Val Asp Phe Lys Trp Leu
        205                 210                 215
caa gca atg gta aaa aat gaa agc ctg ccc ttt tgg gtt cgc ctc ttc 903
Gln Ala Met Val Lys Asn Glu Ser Leu Pro Phe Trp Val Arg Leu Phe
    220                 225                 230
ttt tgg aag caa gtg gca gaa aaa gtc cca ctc cag cca aag cac ttc 951
Phe Trp Lys Gln Val Ala Glu Lys Val Pro Leu Gln Pro Lys His Phe
235                 240                 245                 250
agg att ttg aac cca gtt atc atc aaa gaa act gcc ttc gac atc ctt 999
Arg Ile Leu Asn Pro Val Ile Ile Lys Glu Thr Ala Phe Asp Ile Leu
                255                 260                 265
cag tac tca gag cct cag tca aga ttc tgg ggc cat gat aag aac atc 1047
Gln Tyr Ser Glu Pro Gln Ser Arg Phe Trp Gly His Asp Lys Asn Ile
            270                 275                 280
ccc acg atc ggc gtc att gcc gtt gtc ttg gct aca cat ctg tgt gat 1095
Pro Thr Ile Gly Val Ile Ala Val Val Leu Ala Thr His Leu Cys Asp
        285                 290                 295
gaa gtc agc ctg gca ggc ttt ggc tac gac ctc agt caa ccc agg acc 1143
Glu Val Ser Leu Ala Gly Phe Gly Tyr Asp Leu Ser Gln Pro Arg Thr
    300                 305                 310
cct ctg cac tac ttt gac agt cag tgc atg ggc gcc atg cac tgg cag 1191
Pro Leu His Tyr Phe Asp Ser Gln Cys Met Gly Ala Met His Trp Gln
315                 320                 325                 330
gtc atg cac aat gtg acc aca gag acc aag ttc ctc ctg aag ctc ctc 1239
Val Met His Asn Val Thr Thr Glu Thr Lys Phe Leu Leu Lys Leu Leu
                335                 340                 345
aag gag ggc gtg gtg gag gac ctc agc ggc ggc atc cac tgagaactcg 1285
Lys Glu Gly Val Val Glu Asp Leu Ser Gly Gly Ile His
            350                 355
gaacacggca aacctcaccc agcaccgcag ctgagagcgt ggtgagcagc ctccacaggg 1345
acttcaccct gcagctgctt cgatgtgcag ctagtgtttt caaactccac atttttttta 1405
aaaaaggaaa agaaagaaca acagcaacaa caaaagctct gctctgtgca cctctttgtc 1465
ctatttattt gaagtcagtg ttggattttg cacagttttg taagttaatc ttaagaatgg 1525
gatttggaagg actttcaaa gagaattgta tagtttattg tttttaagg aagtaattta 1585
atttgcagaa actgtacaca cgtactctgc tcaggtgttg aggtgggagg agaggggctt 1645
ctggcccctg gatgatggct gtgatgcccg atactggggt ctgctgctct gtttggtaga 1705
actgatggca gagaaacttc ctgcctccag gataaagggc ttactcatca cctctggcag 1765
ctgctagaca agttcataac cctttctgc tagtccatct gccagctggc ttgcaggact 1825

caggcagggc agctgtcccg gaggctgctg gttggtgagc cactgtcagc tgagtgccgt 1888
gatgttgccc cagggtggaa gaagccacac ttcctacact gtcagggcac ttttaaactt 1948
ctggaggggt gtgtgtgtgt gtgtgtgtgt gtgtgtgtgt gtgtgtgtgt gtgtgtgtgt 2008
gtttattctg cccttccaaa tcatctaagt gttatttaag gcactctgct gtttgtatga 2068
gatggttcat agaaattatg acaaagcctt tgttatccag gccatgggaa gag 2121

<210> 2
<211> 359
<212> PRT
<213> Homo sapiens

<400> 2
Met Arg Arg Pro Ser Leu Leu Ile Lys Asp Ile Cys Lys Cys Thr Leu
1               5                   10                  15
Val Ala Phe Gly Val Trp Leu Leu Tyr Ile Leu Ile Leu Asn Tyr Thr
            20                  25                  30
Ala Glu Glu Cys Asp Met Lys Arg Met His Tyr Val Asp Pro Asp Arg
        35                  40                  45
Ile Lys Arg Ala Gln Ser Tyr Ala Gln Glu Val Leu Gln Lys Glu Cys
    50                  55                  60
Arg Pro Arg Tyr Ala Lys Thr Ala Met Ala Leu Leu Phe Glu Asp Arg
65                  70                  75                  80
Tyr Ser Ile Asn Leu Glu Pro Phe Val Gln Lys Val Pro Thr Ala Ser
                85                  90                  95
Glu Ala Glu Leu Lys Tyr Asp Pro Pro Phe Gly Phe Arg Lys Phe Ser
            100                 105                 110
Ser Lys Val Gln Ser Leu Leu Asp Met Leu Pro Glu His Asp Phe Pro
        115                 120                 125
Glu His Leu Arg Ala Lys Ala Cys Lys Arg Cys Val Val Val Gly Asn
    130                 135                 140
Gly Gly Ile Leu His Gly Leu Glu Leu Gly His Ala Leu Asn Gln Phe
145                 150                 155                 160
Asp Val Val Ile Arg Leu Asn Ser Ala Pro Val Glu Gly Tyr Ser Glu
                165                 170                 175
His Val Gly Asn Lys Thr Thr Ile Arg Met Thr Tyr Pro Glu Gly Ala
            180                 185                 190
Pro Leu Ser Asp Val Glu Tyr Tyr Ala Asn Asp Leu Phe Val Thr Val
        195                 200                 205
Leu Phe Lys Ser Val Asp Phe Lys Trp Leu Gln Ala Met Val Lys Asn

210                 215                 220
Glu Ser Leu Pro Phe Trp Val Arg Leu Phe Phe Trp Lys Gln Val Ala
225                 230                 235                 240
Glu Lys Val Pro Leu Gln Pro Lys His Phe Arg Ile Leu Asn Pro Val
                245                 250                 255
Ile Ile Lys Glu Thr Ala Phe Asp Ile Leu Gln Tyr Ser Glu Pro Gln
            260                 265                 270
Ser Arg Phe Trp Gly His Asp Lys Asn Ile Pro Thr Ile Gly Val Ile
        275                 280                 285
Ala Val Val Leu Ala Thr His Leu Cys Asp Glu Val Ser Leu Ala Gly
    290                 295                 300
Phe Gly Tyr Asp Leu Ser Gln Pro Arg Thr Pro Leu His Tyr Phe Asp
305                 310                 315                 320
Ser Gln Cys Met Gly Ala Met His Trp Gln Val Met His Asn Val Thr
                325                 330                 335
Thr Glu Thr Lys Phe Leu Leu Lys Leu Leu Lys Glu Gly Val Val Glu
            340                 345                 350
Asp Leu Ser Gly Gly Ile His
        355

<210> 3
<211> 17
<212> DNA
<213> Artifical Sequence
<220>
<223> Synthetic DNA as 5'-primer

<400> 3
atgaaaagaa tgcacta                                                    17

<210> 4
<211> 17
<212> DNA
<213> Artificial Sequence
<220>
<223> Synthetic DNA as 3'-primer

<400> 4
tcagtggatg ccgctga                                                    17

<210> 5
<211> 18
<212> PRT
<213> Homo sapiens

<400> 5
Leu Leu Lys Leu Leu Lys Glu Gly Val Val Glu Asp Leu Ser Gly Gly
1               5                   10                  15
Ile His

<210> 6
<211> 48
<212> PRT
<213> Homo sapiens

<400> 6
Cys Lys Arg Cys Val Val Val Gly Asn Gly Gly Ile Leu His Gly Leu
1               5                   10                  15
Glu Leu Gly His Ala Leu Asn Gln Phe Asp Val Val Ile Arg Leu Asn
            20                  25                  30
Ser Ala Pro Val Glu Gly Tyr Ser Glu His Val Gly Asn Lys Thr Thr
        35                  40                  45

## Description

### BACKGROUND OF THE INVENTION

The present invention relates to a sialyltransferase and to a DNA encoding the same. More particularly, the present invention relates to an enzyme which synthesizes ganglioside G_{M3} by transferring sialic acid to a galactose residue of lactosylceramide and to a DNA encoding the enzyme.

Human myelogenous leukemia cell line HL-60, which is a cell line that has acquired the ability of infinite proliferation as a result of tumorigenic transformation, is used generally and widely as a model for leukemia cells (Collins, S.J. Gallo, R.C., and Gallagher, R.E., Nature (London), 270, 347-349 (1977); Collins, S.J., Blood, 70, 1223 (1987)). The cell line does not differentiate even after continued cultivation and continues to proliferate while it remains as undifferentiated cells. However, when cultivation is continued with addition of phorbol ester, which is widely used as a differentiation inducer, the cell line stops the proliferation of cells and takes an appearance similar to that of monocytes or macrophages. This indicates that differentiation has been induced. It has been reported that during this process, the amount of G_{M3}, which is a kind of ganglioside, increases considerably (Nojiri, E., Takaku, F., Tetsuka, T., and Saito, M., Blood, 64, 534-541(1984)), and when the ganglioside G_{M3} is added exogenously, the cell line shows the same change as that observed with the addition of phorbol ester, i.e., the cells undergo monocytic differentiation (Saito, M., Terui, Y., and Nojiri, H., Biochem. Biophys. Res. Commun., 132, 223-231 (1985)). Also, in has been proved that in this differentiation process, G_{M3} itself has an activity of inducing differentiation (Nojiri, H., Takaku, F., Miura, Y., and Saito, M., Proc. Natl. Acad. Sci. U.S.A., 83, 782-786 (1986)), and that chemically synthesized G_{M3} also induces differentiation (Sugimoto, M. and Ogawa, T., Glycoconj. J., 2, 5-9 (1985); Saito, M., Nojiri, H., Ogino, H., Yuo, A., Ogura, H., Itoh, M., Tomita, K., Ogawa, T., Nagai, Y., and Kitagawa, S., FEBS Lett., 271, 85-88 (1990)).

On the other hand, it has been elucidated that sialic acid-containing glycolipids, in particular ganglioside, bear important functions in various biological phenomena and not only its functions but also its biosynthesis are being clarified. In vertebrates, many gangliosides (ganglio-series gangliosides) have a common precursor, G_{M3}, which has the simplest structure among major gangliosides and the G_{M3} synthesis affords a basis for the biosynthesis of gangliosides which have major functions.

As described above, ganglioside G_{M3} itself participates in the proliferation/differentiation of cells and tissues and it is suggested that the ganglioside G_{M3} is a precursor for a group of higher gangliosides having various functions in vertebrates.

G_{M3} has been considered to be synthesized from lactosylceramide by transfer of sialic acid to the galactose residue in lactosylceramide by CMP-sialic acid:lactosylceramide sialyltransferase (CMP-NeuAc: Galβ1-4Glcβ1-1'Cerα2,3-sialyltransferase; SAT-1). However, neither the transferase from human has been isolated nor the genes thereof have been identified.

Enzymes which transfer sialic acid through an α2-3 ketoside bond are described in, for example, Wienstein et al., J. Biol. Chem., 257, 13835 (1982); Gillespie et al., Glycoconj., 7, 469 (1990); Gillespie, W., Kelm, S. and Paulson, JC., J. Biol. Chem., 267, p21001-21010 (1992); Lee, YC., Kojima, N., Wada, E., Kurosawa, N., Nakaoka, T., Hashimoto, T. and Tsuji, S., J. Biol. Chem., 269, p10028-10033 (1994); Kim. YJ., Kim, KS., Kim, SH., Kim, CH., Ko, JH., Choe, IS., Tsuji, S. and Lee, YC., Biochem. Biophys. Res. Commun., 228, p324-327 (1996); and JP-A 5-336963. However, none of the enzymes is known to be involved in the synthesis of G_{M3} or shows an enzyme activity of transferring sialic acid to lactosylceramide through an α2-3 ketoside bond. Sandhoff, K. et al. presume that α2-3 sialyltransferase (SAT4) is identical with the enzyme which synthesizes G_{M3} (J. Biol. Chem., 268, 5341 (1993)). However, this is a presumption based on an indirect method, which fails to support that the enzymes are identical to each other as a substance.

In spite of various attempts which have been made in order to elucidate and control its biosynthesis according as the clarification of importance of ganglioside G_{M3} proceeds, the above-mentioned sialyltransferase, which relates closely to the synthesis of G_{M3}, has not been isolated yet because of difficulty in preparing the enzyme protein and, hence, neither its gene expression control mechanism has been clarified yet nor its proteo-chemical or enzymological analysis has been performed successfully.

### SUMMARY OF THE INVENTION

As a result of intensive investigation with view to elucidating the control mechanism of cell differentiation by carrying forward studies on gene expression control mechanism of and proteo-chemical and enzymological analyses of the above-mentioned sialyltransferase, the present inventors have been successful in isolating cDNA having a nucleotide sequence encoding the sialyltransferase which participates in the above-mentioned G_{M3} synthesis, by using an expression cloning method and based on the nucleotide sequence, they have clarified the structure of the above-mentioned sialyltransferase. As a result, it revealed that the enzyme is low in homology with the known sialyltransferase and is believed to be a new enzyme, differing from the α2-8 sialyltransferase, with which the identity was presumed by Sandhoff, K. supra.

Accordingly, the present invention provides a sialyltransferase having the following properties and a DNA having a nucleotide sequence encoding it.
(1) Activity:
   The sialyltransferase transfers sialic acid from a sialic acid donor selectively to a 3-hydroxyl group of a galactose residue contained in lactosylceramide as a sialic acid acceptor to produce ganglioside G_{M3}.
(2) Optimal reaction pH:
   6.0 to 7.0
(3) Activation:
   The activity increases at least 1.5 times with 10 mM of Mn²⁺ as compared with the case in the absence thereof.

Also, the present invention provides a sialyltransferase having the above-mentioned activity and having an amino acid sequence shown by SEQ ID No: 5 on the C-terminal of the amino acid sequence thereof and a DNA encoding it as well as a sialyltransferase having the above-mentioned activity and having an amino acid sequence shown by SEQ ID NO: 6 and a DNA encoding it.

The sialic acid donor is preferably cytidine 5-monophosphsate-sialic acid (CMP-sialic acid).

The above mentioned enzymes and DNAs are preferably those derived from a mammal, most preferably those derived from human.

The present invention also provides a sialyltransferase comprising the polypeptide (a) or the polypeptide (b) below and a DNA encoding it.
(a) A polypeptide having an amino sequence shown by SEQ ID NO: 2.
(b) A polypeptide having an amino acid sequence (a) above, which has therein substitution, deletion, insertion or rearrangement of one or a few amino acid residues, said sialyltransferase having an enzyme activity of transferring sialic acid from a sialic acid donor selectively to the 5-hydroxyl group of galactose residue contained in lactosylceramide as a sialic acid acceptor to produce ganglioside G_{M3}.

Specific examples of the DNA of the present invention include a DNA having a nucleotide sequence encoding all the amino acid sequence shown by SEQ ID NO: 2 or a DNA having partial sequences thereof, for example, DNA having a nucleotide sequence shown by SEQ ID NO: 1.

Further, the present invention provides a polypeptide comprising all or part of the polypeptide of sialyltransferase encoded by the nucleotide sequence of the above-mentioned DNA. From the polypeptide, a transmembrane domain may be deleted.

The phrase "encoding an enzyme" as used herein refers to encoding the polypeptide of the enzyme. Also, herein, the sialyltransferase of the present invention which has an enzyme activity of transferring sialic acid from a sialic acid donor selectively to the 3-hydroxyl group of the galactose residue contained in lactosylceramide as a sialic acid acceptor to produce ganglioside G_{M3} is also described as "sialyltransferase-1" or "SAT-1" for convenience's sake.

According to the present invention, a DNA of α2-3 sialyltransferase (SAT-1) which synthesize from lactosylceramide, ganglioside G_{M3} that induces cell differentiation. According to the present invention, α2-3 sialyltransferase, i.e., G_{M3} synthase, can be obtained easily by the use of the above-mentioned DNA.

Since the DNA encoding SAT-1 is provided by the present invention, the elucidation of expression mechanism thereof will give an expectation for elucidation of the mechanism of cell differentiation.

### BRIEF DESCRIPTION OF THE DRAWINGS

Fig. 1 is a schematic view showing the structure of α2-3 sialyltransferase (SAT-1) of the present invention, in which Δ indicates an N-glycosylation site presumed from the amino acid sequence and TM indicates a transmembrane domain presumed from the amino acid sequence.

Fig. 2 is a diagram comparing the amino acid sequence of a sialyl motif (L and S) region of SAT-1 with a sialyl motif region of another sialyltransferase; the marks "*" under the sequences indicate common sequences appearing in the sialyl motif of other sialyltransferases, the marks "*" above the sequences indicate the part of SAT-1 that contains the amino acids identical with the amino acids in the common sequences of the sialyl motif, and the marks "-" above the sequences indicate the part that contains amino acids different from those in the common sequences of the sialyl motif.

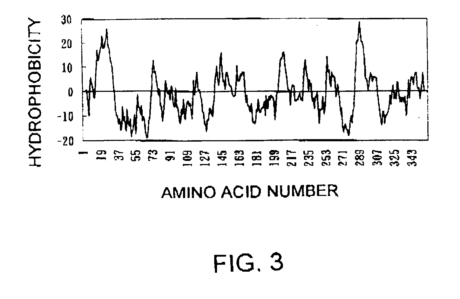

Fig. 3 is a graph illustrating hydropathy-plot of an amino acid sequence of SAT-1 deduced from a nucleotide sequence of the DNA of the present invention.

### DETAILED DESCRIPTION OF THE INVENTION

### [1] DNA encoding sialyltransferase-1 of the present invention (DNA of the present invention)

The DNA of the present invention includes DNAs having nucleotide sequences encoding sialyltransferases containing various polypeptides, the sialyltransferases having the following physico-chemical properties:

### (1) Activity:

The sialyltransferase selectively transfers sialic acid from a sialic acid donor to a 3-hydroxyl group of a galactose residue contained in lactosylceramide as a sialic acid acceptor, to produce ganglioside G_{M3}. In other words, the enzyme does not transfer substantially sialic acid to a position except for a 3-hydroxyl group of a galactose residue of the above-mentioned sialic acid acceptor. The sialic acid acceptor is preferably CMP-sialic acid.

### (2) Optimal reaction pH:

This enzyme has high sialic acid transferring activity within the range of an enzyme reaction mixture pH of from 6.0 to 7.0 as measured by the enzyme activity assay method described in the examples below.

### (3) Activation:

The activity of sialyltransferase increases at least 1.5 times in the presence of 10 mM of Mn²⁻ as compared with the case in the absence thereof.

Further, the DNA of the present invention includes DNAs which have nucleotide sequences encoding various polypeptides of sialyltransferases containing polypeptides which have the activity (1) above and having an amino acid sequence shown by SEQ ID NO: 5 on the C-terminal of the amino acid sequence thereof as well as those DNAs which have nucleotide sequences encoding various polypeprides of sialyltransferases containing polypeptides which have the activity (1) above and having an amino acid sequence shown by SEQ ID NO: 6. The amino acid sequence shown by SEQ ID NO: 6 is a sequence which corresponds to a sialyl motif existing in the sialyltransferase and which usually exists in the part corresponding to the amino acid numbers 136-183 in the amino acid sequence shown by SEQ ID NO: 2 in the amino acid sequence of the polypeptide of the sialyltransferase.

The DNA of the present invention also includes those encoding the polypeptides (a) or (b) below and their nucleotide sequence is not limited particularly as far as they encode those polypeptides.
(a) A polypeptide having an amino sequence shown by SEQ ID NO: 2.
(b) A polypeptides having the amino acid sequence (a) above, which has therein substitution, deletion, insertion or rearrangement of one or a few amino acid residues and having an enzyme activity of transferring sialic acid from a sialic acid donor selectively to a 3-hydroxyl group or a galactose residue contained in lactosylceramide as a sialic acid acceptor to produce ganglioside G_{M3}.

In other words, the amino acid sequence shown by SEQ ID NO: 2 may have therein substitution, deletion, insertion or rearrangement of one or a few amino acid residues that do not substantially impair the activity of transferring sialic acid from a sialic acid donor selectively to a 3-hydroxyl group of a galactose residue contained in lactosylceramide as a sialic acid acceptor to produce ganglioside G_{M3}. The DNA of the present invention includes DNAs having any of substitution, deletion, insertion and rearrangement in their nucleotide sequence, encoding such polypeptides having therein any of substitution, deletion, insertion and rearrangement in their amino acid sequence. The term "a few amino acid residues" as used herein refers to the number of amino acids that may cause variations to the extent that the activity of the enzyme is not lost. In the case of a polypeptide consisting of 360 amino acid residues, for example, it means about 20 or less. The activity of the enzyme can be measured without difficulty by a known method (JP-A 7-327678), by changing cDNA to be introduced into host cells and a substrate for the enzyme and since one skilled in the art can practice with ease, for example, by the method specifically described herein, the substitution, deletion, insertion or rearrangement of one or a few amino acid residues that does not substantially impart the target activity can be readily selected by using the presence or absence of the enzyme activity as an indicator. The substitution, deletion, insertion or rearrangement in the nucleotide sequence can be introduced into a DNA by synthesizing a sequence having a restriction enzyme-cleaved end on each terminal and containing bolt sides of the mutation point, i.e., substitution, deletion, insertion or rearrangement, followed by replacing this for the corresponding part of the nucleotide sequence of a non-mutated DNA. Alternatively, site-specific mutagenesis (Kramer, W. and Frits, H. J., Meth. in Enzymol., 154, 350 (1987); Kunkel, T. A. et al., Meth. in Enzymol., 154, 367 (1987)) and the like can be used to introduce substitution, deletion, insertion or rearrangement into a DNA. Also, the DNA encoding a polypeptide having substitution, deletion, insertion or rearrangement of one or a few amino acid residues that does not substantially impair the activity in the amino acid sequence shown by SEQ ID NO: 2, may be obtained as a homologous or alleic variant.

Specifically, the DNA of the present invention includes DNAs having nucleotide sequences encoding all the amino acid sequence shown by SEQ ID NO: 2 or DNAs having partial nucleotide sequences thereof. These DNAs are preferred but the present invention is not limited thereto. The term "DNAs having partial nucleotide sequences" as used herein refers to, for example, those DNAs which hybridize with DNA encoding a polypeptide of sialyltransferase-1 so that they can be used as a probe for detecting the DNA of sialyltransferase-1; which encode the polypeptides having sialyltransferase-1 activity; or which encode the polypeptides having antigenicity similar to that of sialyltransferase-1. The hybridization referred to above may be performed under stringent conditions by a method which is conventionally used for hybridizing DNA or RNA with DNA, such as screening. For example, the conditions used in screening DNA or the like include prehybridizing a target DNA in a solution containing 50% formamide, 5× SSPE (sodium chloride/sodium phosphate/EDTA buffer), 5× Denhardt's solution, 0.5% SDS, and 50 µg/ml of denatured salmon sperm DNA, adding to the solution ³²P-labeled DNA of the present invention (for example, DNA having a nucleotide sequence shown by SEQ ID NO: 1), hybridizing it at 42°C for 16 hours, and then washing it sequentially with 1×SSPE, 1% SDS, 0.1×SSPE, and 0.1% SDS at 55°C. Although generally hybridization is performed mostly under the above-mentioned conditions, one skilled in the art can perform similar hybridization by changing the composition of each solution and conditions aiming at similar hybridization and, hence, the present invention is not limited to the above-described conditions as far as the conditions used enable one to obtain similar effects.

More specifically, the DNA of the present invention includes DNAs having the whole nucleotide sequence shown by SEQ ID NO: 1 or partial sequences thereof, which are preferred. Specific examples of these DNAs include a DNA having a nucleotide sequence of base numbers 202-1278 in the sequence shown by SEQ ID NO: 1.

In the nucleotide sequence shown by SEQ ID NO: 1, the 5'-terminal portion of the open reading frame of cDNA of sialyltransferase-1 contains three in-frame ATG codons. The nucleotide sequences around the three ATG codons conserve each a purine base at the -3 position. This satisfies the Kozak's finding on efficient translation (Kozak, M., (1986) Cell, 44, 283-292) so that it is possible that any of the ATG codons functions as an initiation codon.

In the meantime, β-1,4-galactosyltransferase is known to contain two in-frame ATG codons (Nakazawa, K. et al. (1988) J. Biochem., 104, 165-168; Shaper, N. et al. (1988) J. Biol. Chem., 263, 10420-10428). Also, Shaper et al. showed that in the case of β-1,4-galactosyltransferase, translation starts at two sites, resulting in that the enzyme is synthesized in both longer and shorter forms. Further, Lopez et al. presented the evidence suggesting that the longer form preferentially targets membrane while the shorter form exists mainly in Golgi apparatus (Lopez, L. et al. (1991) J. Biol. Chem., 266, 15984-15591). Similarly, in the case of the sialyltransferase, there is the possibility that plural ATG codons serve as an initiation codon. This is not certain yet. However, no matter how ATG codon may be an initiation codon, it is common that the polypeptide of the above-mentioned sialyltransferase-1 is encoded. Therefore, DNAs having nucleotide sequences starting with the second and third ATG codons, respectively are also embraced by the present invention.

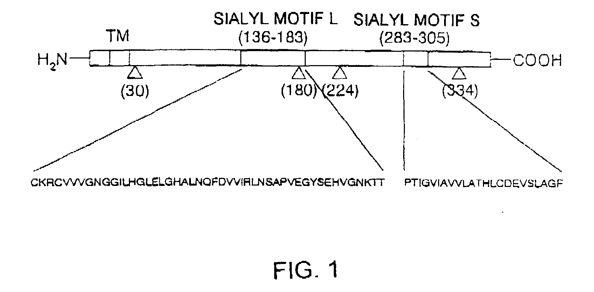

From a single open reading frame starting with the first ATG codon in the sequence shown by SEQ ID NO: 1 is deduced a protein which consists of 359 amino acid residues, has a molecular weight of 41,244 Da, and contains four sites that can be an N-glycosylation site. Fig. 1 is a graph illustrating hydropathy plot prepared from this amino acid sequence. From Fig. 1 it can be seen that there exists in the sequence a 14 residue-long continuous, remarkably hydrophobic part ranging from the 16th to 29th amino acid residues counting from the N-terminal, which suggests that the protein has a transmembrane domain.

It will be readily understood by one skilled in the art that the DNA of the present invention also includes DNAs having different nucleotide sequences by degeneracy of genetic codes.

Further, the DNA of the present invention include DNAs or RNAs complementary to the DNA of the present invention. Furthermore, the DNA of the present invention may be of a single strand of only a coding strand which encodes SAT-1 or a double strand of the single strand and a DNA or RNA strand having a nucleotide sequence complementary thereto.

Also, the DNA of the present invention may have a nucleotide sequence over the whole encoding region which encodes the whole peptide of SAT-1 or a nucleotide sequence encoding only a part of the polypeptide of SAT-1.

Now, generally, mammal sialyltransferases are known to have high homology in their amino acid sequence. The polypeptide which the DNA of the present invention encodes is expected to have a homology of about 65% or more in the species. Therefore, polypeptides having high homology with the polypeptides encoded by the DNAs of the present invention specifically disclosed herein and DNAs encoding such polypeptides (such as homologous or alleic variants) are also embraced by the present invention. As described above, the polypeptide of SAT-1 has a transmembrane domain. The part of the polypeptide of SAT-1 that has lost the region starting from the N-terminal corresponding to the N-terminal inside the membrane and containing the region having the transmembrane domain is also embraced by the present invention. Such a polypeptide includes, for example, an amino acid sequence of amino acids numbers 38 to 359 in the amino acid sequence shown by SEQ ID NO: 2.

### [2] Method of producing the DNA of the invention

Hereafter, a method of producing the DNA of the present invention will be explained in detail. As the amino acid sequence of the polypeptide of SAT-1 has been clarified by the present invention, it is possible to obtain the DNA by amplification from chromosomal DNA or m-RNA by a PCR method (polymerase chain reaction method) using an oligonucleotide primer prepared based on the amino acid sequence. Alternatively, the DNA of the present invention can also be produced by an expression cloning method, particularly the method which comprises the following steps.
(1) Cancer cells are treated with a differentiation inducing agent to cause differentiation.
(2) A cDNA library is prepared from differentiated cancer cells and is introduced into host cells.
(3) Host cells that have expressed ganglioside on the cell membrane are screened.
(4) The screened host cells are sorted to enrich the library.
(5) The introduced gene is excised from the enriched library.

The whole length of cDNA of the above-mentioned SAT-1 is normally selected by means of screening.

Hereafter, an example of the method of producing the DNA of the present invention will be explained more specifically.

### (1) Differentiation induction in cancer cells

Cancer cells are preferably anchorage independent cells. Such cancer cells include blood cell lymphoma and leukemia cells, which are preferred. As such cells are preferred mammalian-derived cells, for example, human-derived HL-60 (ATCC CCL240), MOLT-4 (ATCC CRL 1582), and U937 (ATCC CRL1593) and mouse-derived M1 (ATCC TIBl92) and fresh myelogenous leukemia cells can also be used. Among such cancer cells, most preferred are human-derived cells and HL-60 cells are particular preferred since differentiation induction is readily performed. Differentiation is induced by cultivating the cultivated cancer cell line for 20 hours or more, preferably for about 24 to 48 hours, after adding a differentiation inducing agent to the cancer cell line. Cultivation may be performed under conditions that are suited for the cells used. Usually, as general cell culture conditions, there can be used conditions of 5-7 vol% of CO₂ and 95-93 vol% of air at 37-38°C. As the differentiation inducing agent, there can be used, for example, phorbol ester (12-O-tetradecanoyl phorbol ester (TPA) etc.), dimethyl sulfoxide (DMSO), retinoic acid (RA), and 1α,25-dihydroxyvitamin D₃ (1α,25(OH₂D₃)) and the like. Although the present invention is not limited to the use of a particular one, preferred among them is TPA since it has relatively uniform differentiation inducing activity toward many leukemia cell lines. When HL-60 is used as a cancer cell and TPA is used as a differentiation inducing agent, 48 hour cultivation in the presence of TPA in an amount of about 24 nM leads to the differentiation of HL-60 into monocyte/macrophage like cells, showing morphological changes.

### (2) Construction of cDNA from differentiated cancer cells

### 1) Preparation of RNA from differentiated cancer cells

The cancer cells of which dffferentiation is induced in above (1) are collected by centrifugation preferably at 500 to 2,000 × g and total RNA is prepared from the cells by a known method, for example, a guanidine thiocyanate/CsC1 method (Kingston, R. E., (1991) in Current Protocols in Molecular Biology, Suppl. 14, Unit 4.2, Green Publishing Associates and Wiley Interscience, New York). From the total RNA thus obtained is purified poly(A)⁺RNA by oligo-(dT)cellulose column chromatography or the like.

### 2) Construction of cDNA from poly(A)⁺RNA

Reverse transcription PCR using the above-mentioned poly(A)⁺RNA as a template and also an oligonucleotide primers allows amplification of cDNA derived from the cancer cells. PCR may be performed In the same manner as a conventional method. Specific method thereof may be as follows. Namely, a buffer solution (final volume 20 µl) containing 1 µl of poly(A)⁺RNA, 100 pmol of oligo-(dT), 100 pmol each of random oligonucleotide primers, 500 µl each of 4 kinds of deoxyribonucleoside triphosphates, 200 units of M-MLV reverse transcriptase (Gibco BRL), 1 mM dithiothreitol (DTT), 120 units of RNase (ribonuclease) inhibitor (manufactured by TAKARA SHUZO Co., LTD.) was incubated at 50°C for 60 minutes to synthesize a cDNA primary strand. Next, a reaction mixture (final volume 50 µl) containing 5 µl of the above-mentioned reverse transcriptase reaction mixture, 100 pmol each of random oligonucleotide primers, 250 µM each of 4 kinds of deoxyribonucleoside triphosphates, and 1.25 units of Taq polymerase was incubated by repeating 35 cycles of 95°C for 1 minute, 46 to 62°C for 1 minute, and 72°C for 2 minutes.

The cDNA of cancer cells thus obtained is made to be held by an expression vector and introduced into host cells for screening the host cells. As the host cells, there can be used any cells as far as they are cells of a mammalian-derived cell line which are lactosylceramide-positive. Examples of such include human Namalwa cells (Hosoi et al.: Cytotechnology, 1, 151 (1988)), Chinese hamster-derived CHO cells (ATCC CCL61, etc.), monkey-derived COS cells (ATCC CRL1650, etc.), mouse-derived 3LL cells (Taniguchi, S., Shinshu University Aging Adaptation Research Center)) and so on. However, since the detection of SAT-1 enzyme activity can be made easier in the present invention, those cultivated cells which are further G_{M3}-negative are preferred. Examples of such cells include 3LL-HK46 cell (Inokuchi, J., (Seikagaku Corporation)), a mutant of 3LL cell, which is preferred. The expression vector includes pCEV18 (Maruyama, K. (donated from Tokyo University, Medical Science Research Institute, now Tokyo Medical Dental University), pCXN2 (Niwa, H., Yamamura, K. and Miyazaki, J. (Gene, 108, p193-200 (1991)), pFLAG-CMV-2 (manufactured by Eastman Kodak), pAGE107 (Miyaji et al., Cytotechnology, 3, 133 (1990)), pAS3-3 (JP-A 2-227075), pAMoERC3Sc(JP-A 5-336963), pcD2 (Chen, C. et al., Mol. Cell. Biol., 7, 2745-2752 (1987)) and the like and can be selected appropriately taking into consideration the host cell to be used. For example, when 3LL-HK46 is used as a host cell, it is preferred that pCEV18 be used as an expression vector. Introduction, into a vector, of the PCR product prepared based on poly(A)⁺RNA of a cancer cell as described above is performed by a method selected from known methods which is suited for the vector to be used.

### 3) Introduction of cDNA library into a host cell

The cDNA library constructed by the above-mentioned method is transfected to host cells by a known technique. Specifically, there can be cited, for example, an electroporation method (Miyaji, et al., Cytotechnology, 3, 133 (1990)), a calcium phosphate method (JP-A 2-227075), and a lipofection method (Philip, L. F. et al., Proc. Natl. Acad. Sci. USA, 84, 7413 (1987)), and selected suitably. However, the electroporation method is preferred. When cDNA encoding SAT-1 is detected, it is also possible to transfect, in advance or simultaneously, to host cells, DNA encoding human α2-8 sialyltransferase (JP-A 7-327678, etc.), an enzyme which synthesizes G_{D3} that is known to be synthesized from G_{M3}, express on cell membrane, and can be detected with ease, directly from G_{M3}. Such a pretransfection or simultaneous transfection is preferred. Therefore, when the cDNA library constructed by using pCEV18, for example, as a vector is introduced into 3LL-HK46 cells having no G_{D3} synthesis pathway as a host cell, pCEV18 holding the cDNA library may be transfected to normal 3LL-HK46 cells directly or to 3LL-ST28, a recombinant made by introducing in advance the cDNA of α2-8 sialyltransferase into 3LL-HK46 cells using an eukaryote expression vector such as pCEV18. Also, pCEV18 containing the cDNA library may be transfected to 3LL-HK46 cells simultaneously with the vector into which the cDNA of α2-8 sialyltransferase has been introduced.

### (3) Detection of host cells expressing ganglioside

Host cells into which cDNA library has been introduced are cultivated under generally used cell culture conditions. After at least 24 hours, preferably after 36 to 48 hours, from the introduction of cDNA, the host cells are stained by immuno-staining using an anti-ganglioside antibody or a lectin which bonds to ganglioside. The staining method using antibodies is more accurate and preferred. For example, when 3LL-HK46 cell is used as a host cell, the expressing cells are detected by using an antibody which recognizes G_{M3} that has been expressed on the cell membrane, for example, anti-G_{M3} monoclonal antibody M2590 (the monoclonal antibody which L612 (ATCC CRL10724)) produces: J. Biol. Chem., 260, 13328-13333 (1985)). The immuno-staining can be performed by a conventional method. When the above-mentioned 3LL-ST28 is used as a host cell, for example, G_{D3} which is synthesized from the G_{M3} produced upon the introduction of the DNA of the present invention is detected. Immuno-staining for detecting G_{D3} may be performed by a conventional method generally used (JP-A 2-327678). In this case, the primary antibody to be used is not limited particularly as far as it is an antibody which recognizes G_{D3}. However, monoclonal antibodies are preferred and examples of which include anti-G_{D3} monoclonal antibody R24 (monoclonal antibody which a hybridoma (ATCC HB8445) produces: Cancer Res., 49, p191-196 (1989)), which is preferred. Specifically, the immuno-staining method using the above-mentioned generally employed antibodies is performed as follows. Namely, the host cells (1×10⁵ cells) after the above-mentioned cultivation are washed about 2 or 3 times by centrifugation in a BSA solution (0.1% BSA PBS(+)) and the cells are suspended in 100 µl of the BSA solution containing the primary antibody. After allowing the suspension to react under ice cooling for 30 minutes, the cells are washed with the above-mentioned BSA solution 2 times or so. Further, in 100 µl of a BSA solution containing 1 µl of FITC-labeled secondary antibody against the primary antibody, the cells are left to stand for 30 minutes under ice cooling for reaction. The cells are washed with a BSA solution once and those cells which show strong fluorescence are detected by using a flow cytometer (FACScalibur: manufactured by Becton Dickinson). Cells showing strong fluorescence, for example, 5% of the total cells, are selected by a cell sorter and plasmid DNA is extracted therefrom. The extraction of plasmid DNA from the host cells is performed by a conventional method.

### (4) Sorting of SAT-1 cDNA and obtaining of cDNA

The plasmid DNA obtained by the above-described operation is transfected to a suitable host cell line and the procedure of immuno-staining with the anti-G_{M3} antibody and recovery of strong fluorescence-showing cells in an amount of 5% of the total cells by using a flow cytometer, for example, is repeated twice or more to enrich the target cDNA by sorting. The host cell used for the sorting is preferably cultivated mammalian cells, of which 3LL-HK46 is particularly preferred. The vector to be used is not limited particularly and any expression vectors for mammalian cells may be used, but pCEV18 is preferred. The above-mentioned vector holding the target cDNA enriched by sorting is transfected to mammalian-derived cultivated cells lacking G_{D3} synthesis pathway, such as 3KK-HK46, simultaneously with the expression vector made by introducing the above-mentioned human α2-8 sialyltransferase cDNA by a known method into an expression vector for mammalian cells, such as pBKCMV (manufactured by STRATAGENE CO.) and detection by immuno-staining and a flow cytometer is conducted in the same manner as described above to obtain cells that show strong fluorescence in an amount of 5% of the total cells. From these cells is extracted plasmid DNA by a conventional method. The cDNA excised by a conventional method from the plasmid DNA is used to transform *E. coli* DH10B (manufactured by GIBCO CO.) therewith, and the transfected *E. coli* cells are inoculated so that 100 colonies per well can be formed, followed by sib selection to finally obtain a clone, pCEV4C7, containing an insert (4C7) of about 2.1 kbp.

### (5) Determination of the nucleotide sequence of cDNA 4C7 encoding SAT-1

The nucleotide sequence of the cDNA obtained as described above, as is or after subcloning in a suitable plasmid such as pCRII, is determined by a conventional method.

The nucleotide sequence of the SAT-1-encoding cDNA determined as described above and amino acid sequence deduced from the nucleotide sequence are shown by SEQ ID NO: 1 and the amino acid sequence alone is shown by SEQ ID NO: 2.

Further, the DNA encoding the polypeptide of SAT-1 which lacks a transmembrane domain, i.e., which is in the form of solubilized protein can be obtained as follows. Namely, based on the nucleotide sequence shown by SEQ ID NO: 1 is prepared a primer selected to have a truncated form at the N-terminal side of the polypeptide of the enzyme, and the target DNA is amplified by a PCR method using the cDNA of cloned SAT-1 as a template. For example, when a DNA encoding the polypeptide of a truncated form that lacks 37 amino acid residues at the N-terminal is to be obtained, an oligonucleotide primer is synthesized based on the nucleotide sequence existing at the 3'- and 5'-terminals of the target nucleotide sequence, for example. An oligonucleotide primers having nucleotide sequences are shown by SEQ ID NO: 3 and SEQ ID NO: 4, respectively, for example, may be used as 5'- and 3'- primers, respectively, in order to perform PCR. Then, the target DNA can be obtained from the amplified PCR product, after purification, if desired.

### [3] Polypeptide comprising the whole or part of SAT-1 polypeptide encoded by the nucleotide sequence of the DNA of the present invention

The present invention provides polypeptides which comprise the whole or part or the above-mentioned SAT-1 polypeptide encoded by the DNA of the present invention. The "part" of the polypeptide as used herein means a part which has a kind of activity or function such as having sialyltransferase-1 activity or having antigenicity. The polypeptide may be single or fused with one or more other polypeptides. The polypeptide may also lack a transmembrane domain.

The polypeptide may be with or without a sugar chain. The kind of sugar chain is not limited particularly.

Such a polypeptide can be obtained by, for example, the production method as described below. Determination of presence or absence of the above-mentioned activity or function can be practiced by changing the cDNA to be introduced into host cells and the substrate for tne enzyme in the assay of enzyme activity as described in JP-A 7-327678 and can be performed with ease by one skilled in the art based on, for example, the method described herein specifically.

### [4] Production method for SAT-1 polypeptide utilizing the DNA of the present invention

The SAT-1 polypeptide can be produced by cultivating cells transformed with the above-mentioned DNA of the present invention in a suitable medium, allowing the cells to produce and accumulate in the culture the polypeptide encoded by the DNA of the present invention, and collecting the SAT-1 polypeptide from the culture.

The cells transformed with the DNA of the present invention can be obtained by inserting a fragment of the DNA of the present invention into a known expression vector to construct a recombinant plasmid and conducting transformation with the recombinant plasmid. Examples of the cells which can be used include prokaryotic cells such as *E. coli* and eukaryotic cells such as mammalian cells. When prokaryotic cells such as *E. coli* are used, there occurs no addition of sugar chain to the SAT-1 polypeptide to be produced by the expression of the DNA of the present invention and, hence, pure SAT-1 polypeptide can be obtained. On the other hand, when eukaryotic cells such as mammalian cells are used, addition of sugar chain(s) to the SAT-1 polypeptide produced by the expression of the DNA of the present invention occurs. Therefore, polypeptides can be obtained in the same form as ordinary SAT-1 which contains also a sugar chain.

In this production method, a host-vector-system usually used in the production of proteins may be used. While it is preferred to use a combination of mammalian-derived cultivated cell, such as 3LL-HK46 cell, 3LL-ST28 cell or COS-1 cell, and an expression vector for mammalian cells, such as pCEV18, pME18S (Maruyama et al., Med. Immunol., 20, 27 (1990)), the present invention is not limited thereto. The medium and cultivation conditions may be selected suitably depending on the host cell to be used.

While the DNA of the present invention may be expressed over the whole length thereof, it may be suppressed as a fused polypeptide with another polypeptide. Also, a part of the DNA of the present invention may be expressed as a partial polypeptide.

A specific example of construction of recombinant plasmid which expresses the above-mentioned fused polypeptide is by the following method. Namely, the DNA of the present invention is incorporated into a vector constructed so that a gene introduced into a plasmid such as pGIR201protA (Kitagawa, H. and Paulson, J. C., J. Biol. Chem., 269, 1394-1401 (1994)) can be expressed as a fused protein by a conventional method to construct a vector having genes for plural proteins on the same reading frame. Then, from the vector is excised NheI fragment, which encodes a fused protein, and the fragment is ligated to a suitable vector such as pCEV18 by the same operation as described above.

The polypeptide of the present invention can be collected from the culture by a known purification method for polypeptides. Specifically, there can be used affinity chromatography using a Sepharose column to which lactosylceramide or CMP-sialic acid, for example is bonded. When the DNA of the present invention is expressed as a fused polypeptide, the culture of the host cell can be purified by affinity chromatography using a column to which a substance is bonded having high affinity for the polypeptide fused with SAT-1, such as antibody, in addition to the above-mentioned affinity column. For example, a linker having an amino acid sequence which a specified proteolytic enzyme can recognize and cleave may be incorporated in advance between the SAT-1 and the polypeptide of other protein in the fused polypeptide. This allows the cleavage to occur at the linker site of the fused polypeptide after purification thereof so that SAT-1, whose purification has been difficult heretofore can be obtained. The combination of the specified proteolytic enzyme and the specified sequence which the enzyme recognizes is, for example, a combination of signal peptidase which acts upon the synthesis of proinsulin and signal peptide of insulin. The above-mentioned culture includes a medium and cells in the medium.

The activity of sialyltransferase can be assayed by changing the substrate for the enzyme in a conventional assay method for assaying general ganglioside synthesis (JP-A 7-327678). For example, a suitable amount of the culture or the enzyme purified by the above-described method is added to a reaction mixture containing 100 mM sodium cacodylate, 10 mM manganese chloride, 0.2 mM CMP-radioactive substance-labeled sialic acid, 0.4 mM lactosylceramide, and 0.3% Triton CF-54. The mixture is adjusted to pH 6.5 and incubated at 37°C for 2 hours and the reaction product is developed by a conventional thin layer chromatography and the enzyme activity is determined by using Fujix BAS2000 Bio Imaging Analyzer (manufactured by Fuji Photo Film Co., Ltd.).

### EXAMPLES

The present invention will be described in further detail by examples. However, the present invention is not limited thereto without exceeding the object of the present invention.

### (1) Differentiation induction of HL-60 cell and construction of cDNA

HL-60 cells were cultivated in RPMI-1640 (manufactured by NISSUI PHARM. CO.) containing 24 nM TPA under the conditions of 5 vol% CO₂ and 95 vol% air at 37°C for 48 hours to induce differentiation. The cultivated cells were collected by centrifugation at 1000×g and total RNA was prepared from the collected cells by guanidine thiocyanate-acid-phenol-chloroform method (AGPC method). From differentiated 5×10⁶ cells was obtained about 40 µg of RNA. From the RNA, poly(A)⁺RNA was purified by oligo-(dT) cellulose column chromatography.

The poly(A)⁺RNA was used as a template for reverse transcription reaction to construct a primary strand of DNA, and the DNA in turn was used for synthesizing double-stranded cDNA (Gubber, V. and Hoffman, B. J., Gene, 25, 283 (1983)).

To the double-stranded cDNA was ligated a restriction enzyme BSTX1 adapter and the ligate was introduced into the BSTX1 site of pCEV18 to construct a cDNA library.

### (2) Transfection of cDNA to 3LL-HK46 cells

The above-mentioned cDNA library was introduced into 3LL-HK46 cells by using an electroporation method and the transfected cells were cultivated for 48 hours under the conditions of 5 vol% CO₂ and 95 vol% air at 37°C.

### (3) Detection of host cells expressing ganglioside and preparation of cDNA

The 3LL-HK46 cells after the cultivation were immuno-stained with M2590, anti-G_{M3} antibody, and with FITC-labeled rabbit anti-murine IgG antibody. The stained cells were passed through a flow cytometer (FACScalibur) to detect fluorescence-positive cells. 5% of the cells on the positive side were collected and plasmid DNA was prepared therefrom. Then, the procedures of introduction of cDNA into 3LL-HK46 cells by electroporation, 48-hour cultivation of the transfected cells, immuno-staining, and detection and collection by using a flow cytometer were further repeated twice.

The plasmids finally obtained by this method were introduced into 3LL-HK46 cells together with pBKCMV G_{D3} (a plasmid obtained by introducing the cDNA of human α2-8 sialyltransferase (G_{D3} synthase) to pBKCMV plasmid vector manufactured by STRATAGENE CO.). After cultivating them for 48 hours, the resulting cells were immuno-stained with R24, anti-G_{D3} antibody, and with FITC-labeled rabbit anti-murine IgG antibody, and 5% of the total cells which show strong fluorescence were detected by a flow cytometer and collected.

From these cells was prepared plasmid DNA, which then was transfected to *E. coli* DH10B (manufactured by GIBCO) by electroporation. After repeating the transfection and screening with ampicillin twice, positive colonies were dispensed to each well in a 96-well microplate in an amount of 100 colonies per well. Nine (9) microplates were inoculated with the transfected cells and only one well was selected by sib selection. Then, 2,400 colonies derived from this single well were extended to twenty five (25) 96-well microplates in a population of 1 colony per well and further sib selection gave rise to a positive clone (pCEV4C7). The pCEV4C7 thus obtained was expressed in 3LL-HK46 cells temporarily and flow cytometry analysis was performed using anti-G_{M3} antibody (M2590) in the same manner as described above. 3LL-HK46 cells temporarily expressing pCEV18, as a control, did not express G_{M3} on the cell membrane whereas 3LL-HK46 cells temporarily expressing pCEV4C7 expressed G_{M3} on the cell membrane and fluorescence was detected.

### (4) Determination of nucleotide sequence

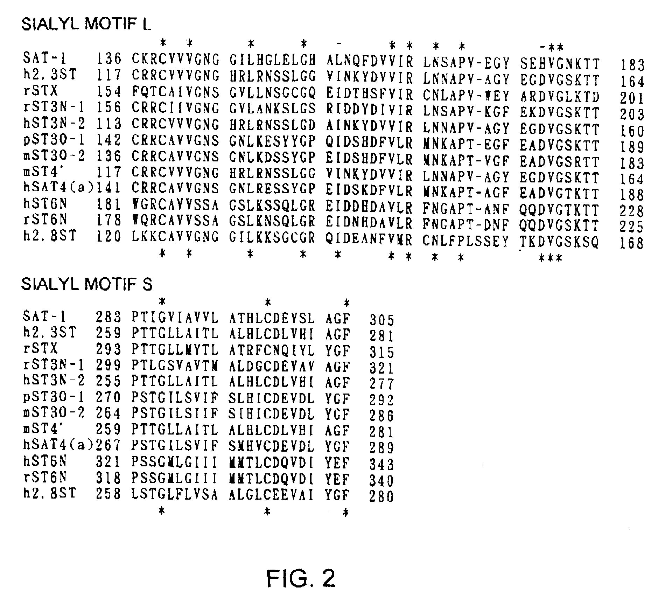

The nucleotide sequence of double-stranded DNA of pCEV4C7 was determined by a dideoxy chain termination method using an autocycle sequencing kit (manufactured by PHARMACIA CO.) and Pharmacia A.L.F. DNA sequencer (manufactured by PHARMACIA CO.). The nucleotide sequence thus determined and an amino acid sequence deduced therefrom are shown by SEQ ID NO: 1 and the amino acid sequence alone is shown by SEQ ID NO: 2. The cDNA insert 4C7 which is contained in pCEV4C7 is of about 2.1 kbp and is revealed to encode a protein (molecular weight 41,244 Da containing 359 amino acid residues starting with a nucleotide at 202 position as a translation initiation point. Fig. 1 is a schematic view which illustrates the structure expected from the amino acid sequence. As a result of hydropathy plot analysis, the amino acid sequence was revealed to correspond to a type-2 membrane protein in which the transmembrane domain (TM in Fig. 1) exists in the region of the 16th to 29th amino acid residues on the N-terminals. Search of this sequence with gene data base in GenBank showed no high homology with any of the data therein. However, with regard to the sialyl motifs (L and S) in the sialyltransferase homologous region existing in the central part and C-terminal region of the sequence for sialyltransferase, relatively high homology was recognized although some substitution was observed (Fig. 2). The sialyltransferases used for comparison were eleven (11) species, i.e., h2,3ST (JP-A 5-336963), rSTX (J. Biol. Chem., 268, 11504-11507 (1993)), rST3N-1 (J. Biol. Chem., 267, 21011-21019 (1992)), hST3N-2 (J. Biol. Chem., 268, 22782-22787 (1993)), pST30-1 (J. Biol. Chem., 276, 21004-21010 (1992)), mST30-2 (Eur. J. Biochem., 216, 377-385 (1993)), mST4' (NCBI Seq. ID 558532), hSAT4(a) (Gycbiology, 5, 319-325 (1995)), hST6N (Nuc. Acids Res., 18, 667 (1990)), rST6N (J. Biol. Chem., 262, 17735-17743 (1987)), h2,8ST (JP-A 7-327678). The results suggest that SAT-1 which is encoded by the insert 4C7 in pCEV4C7 belongs to the sialyltransferase family. Further, the amino acid sequence indicates existence of four consensus sequences of the N-glycosylation site (Δ in Fig. 1), whereas the two sites thereof on the N-terminal side exist near the transmembrane domain and in the sialyl motifs so that these two N-terminal side sites could be less N-glycosylated as compared with the two sites on the C-terminal side.

### (5) G_{M3} synthesis in cells expressing SAT-1 cDNA

pEV4C7 obtained by incorporating the above-mentioned SAT-1-encoding cDNA (4C7) into expression vector pCEV18 was transfected to 3LL-HK46 cells by an electroporation method and the G_{M3} synthase activity of the cells after 48-hour cultivation was assayed by the following method. Namely, 20 µl of a reaction mixture (pH 6.5) containing 0.1 mM CMP-(¹⁴C)-sialic acid (2×10³ CPM), 0.4 mM lactosylceramide, 0.3% (W/V) Triton CF-54, 10 mM MgCl₂, 100 mM sodium cacodylate, 150 µg of the homogenate of 3LL-HK46 cells to which pCEV4C7 was incorporated, and 1 mM sialidase inhibitor (2,3-dehydro-2-deoxy-N-acetylsial ic acid (2,3-dehydro-2-deoxy-NeuAc, manufactured by BOEHRINGER MANNHEIM GMBH) was incubated at 37°C for 2 hours and then 10 µl of methanol was added thereto to stop the reaction. 8 µl of the reaction mixture was charged on a C18 reversed phase thin layer chromatography place (RP-18W HPTLC plate, manufactured by MERCK Co.) and developed with water for 10 minutes. Radioactive substance-labeled reaction products were scrubbed from the original point and G_{M3} was collected therefrom by extraction with 300 µl of chloroform/methanol (1:1, V/V). After the extracts were concentrated to dryness, they were charged on a 60HPTLC plate (manufactured by MERCK CO.) for silica gel thin layer chromatography. After development with chloroform/methanol/0.5% aqueous CaCl₂ solution (55:45:10:, V/V/V), the layer was treated with orcinol sulfate to develop color and measured of radioactivity incorporated into ganglioside using Fujix BAS2000 Bio Imaging Analyzer (manufactured by FUJI PHOTO FILM CO., LTD.). The results revealed uptake of ¹⁴C by ganglioside G_{M3} and G_{M3} synthesis by SAT-1 was detected in the SAT-1 cDNA-transfected cells.

The G_{M3} synthase activity was high at pH 6.0 to 7.0, particularly at around pH 6.5 and increased at least 1.5 times in the presence of 10 mM of Mn²⁺.

## Claims

1. A sialyltransferase having the following physico-chemical properties:
(1) Activity:
transfers sialic acid from a sialic acid donor selectively to a 3-hydroxyl group of a galactose residue contained in lactosylceramide as a sialic acid acceptor to produce ganglioside G_{M3};
(2) Optimal reaction pH:
6.0 to 7.0; and
(3) Activation:
the activity increases at least 1.5 times with 10 mM of Mn²⁺ as compared with the case in the absence thereof.

2. A sialyltransferase having an amino acid sequence shown by SEQ ID NO: 5 on C-terminal of the amino acid sequence thereof, and having the physico-chemical property:
(1) Activity:
transfers sialic acid from a sialic acid donor selectively to a 3-hydroxyl group of a galactose residue contained in lactosylceramide as a sialic acid acceptor to produce ganglioside G_{M3}.

3. A sialyltransferase having an amino acid sequence comprising an amino acid sequence shown by SEQ ID NO: 6, and having the physico-chemical property:
(1) Activity:
transfers sialic acid from a sialic acid donor selectively to a 3-hydroxyl group of a galactose residue contained in lactosylceramide as a sialic acid acceptor to produce ganglioside G_{M3}.

4. The sialyltransferase according to any one of claims 1 to 3, wherein said sialic acid donor is cytidine 5-monophosphate-sialic acid (CMP-sialic acid).

5. The sialyltransferase according to any one of claims 1 to 4, wherein said sialyltransferase is derived from human.

6. A sialyltransferase comprising a polypeptide having an amino acid sequence selected from the group consisting of:
(a) an amino acid sequence shown by SEQ ID NO: 2, and
(b) an amino acid sequence shown by SEQ ID NO: 2, which has therein substitution, deletion, insertion or rearrangement of one or a few amino acid residues, said sialyltransferase having an enzyme activity of transferring sialic acid from a sialic acid donor selectively to a 3-hydroxyl group of a galactose residue contained in lactosylceramide as a sialic acid acceptor to produce ganglioside G_{M3}.

7. A sialyltransferase comprising a polypeptide, wherein said polypeptide having an amino acid sequence which has not less than 65% of homology with an amino acid sequence shown by SEQ ID NO: 2.

8. A sialyltransferase having no transmembrane domain, and said sialyltransferase having an enzyme activity of transferring sialic acid from a sialic acid donor selectively to a 3-hydroxyl group of a galactose residue contained in lactosylceramide as a sialic acid acceptor to produce ganglioside G_{M3}.

9. The sialyltransferase according to claim 8, wherein said sialyltransferase having an amino acid sequence which is defective in a transmembrane domain sequence from an amino acid sequence shown by SEQ ID NO: 2.

10. A sialyltransferase having the amino acid sequence of amino acid numbers 38 to 358 in an amino acid sequence shown by SEQ ID NO: 2.

11. A DNA encoding all or a sialyltransferase-active part of the polypeptide of the sialyltransferase as defined in claim 6.

12. A DNA which has a nucleotide sequence shown by SEQ ID NO: 1.

13. A DNA which has a nucleotide sequence of base numbers 202 to 1278 in a nucleotide sequence shown by SEQ ID NO: 1.

14. A DNA which hybridizes with a polynucleotide having a nucleotide sequence complementary to a nucleotide sequence shown by SEQ ID NO: 1 under stringent conditions.

15. A DNA which hybridizes with a polynucleotide having a nucleotide sequence complementary to a nucleotide sequence of base numbers 202 to 1278 in the nucleotide sequence shown by SEQ ID NO: 1.
